# EUROPÄISCHE PATENTANMELDUNG

(11) **EP 2 777 808 A1**
(43) Veröffentlichungstag der Anmeldung: **17.09.2014**
(21) Anmeldenummer: 13158676.0
(22) Anmeldetag: 12.03.2013
(51) Int. Cl.: B01J 19/00

(54) **Verfahren zur Handhabung deflagrationsfähiger Feststoffe unter reduziertem Druck**

(71) Anmelder: Bayer Technology Services GmbH, 51368 Leverkusen (DE)
(72) Erfinder: Salg, Steffen, 10787 Berlin (DE); Morhenn, Heinrich, Dr., 50629 Köln (DE)
(74) Vertreter: BIP Patents

(57) **Zusammenfassung**

Die Erfindung betrifft ein Verfahren zur Verarbeitung und Handhabung von deflagrationsfähigen Feststoffen und Gemischen, insbesondere zur Verarbeitung deflagrationsfähiger Stoffe in der chemischen und pharmazeutischen Industrie, wobei die Verarbeitung und Handhabung in einer Umgebung unter reduziertem Druck erfolgt.

## Beschreibung

Die Erfindung betrifft ein Verfahren zur Verarbeitung und Handhabung von deflagrationsfähigen Feststoffen und Gemischen, insbesondere zur Verarbeitung deflagrationsfähiger Stoffe in der chemischen und pharmazeutischen Industrie, wobei die Verarbeitung und Handhabung in einer Umgebung unter reduziertem Druck erfolgt.

Die Technische Regel für Anlagensicherheit (TRAS) Nr. 410 definiert eine Deflagration wie folgt:

"Eine Deflagration ist eine Reaktion, die an einer vorgegebenen Stoffmenge örtlich begrenzt auslösbar ist und die sich von dort selbständig durch die gesamte Stoffmenge hindurch in Form einer Reaktionsfront fortpflanzt. Die Fortpflanzungsgeschwindigkeit der Reaktionsfront ist niedriger als die Schallgeschwindigkeit im Stoff. Bei einer Deflagration können große Mengen an heißen Gasen freigesetzt werden, die unter Umständen auch brennbar sind. Die Deflagrationsgeschwindigkeit nimmt mit der Temperatur und in der Regel auch mit dem Druck zu".

Deflagrationsfähige Feststoffe zersetzen sich nach lokaler Einwirkung einer hinreichend starken Zündquelle (Initiierung) auch ohne Anwesenheit von Luftsauerstoff. Im Gegensatz zu einem Brand oder einer Explosion kann eine Deflagration nicht durch Sauerstoffausschluss unterbunden werden. Die aus dem Explosionsschutz bekannte Maßnahme der Inertisierung mit Stickstoff oder anderen inerten Gasen bietet keinen Schutz vor einer Deflagration. Die Verarbeitung unter Vakuum wurde bisher nicht als Schutzmaßnahme für die Verarbeitung und Handhabung von deflagrationsfähigen Stoffen angesehen.

Explosionen sind schnelle Deflagrationen mit sprunghaftem Druck- und Temperaturanstieg. Bei Überschreiten der Schallgeschwindigkeit geht eine Deflagration in eine Detonation über.

Bei den deflagrationsfähigen Stoffen handelt es sich zumeist um organische oder anorganische Verbindungen in fester Form. Insbesondere neigen zur Deflagration organische Verbindungen mit funktionellen Gruppen wie Kohlenstoff-Kohlenstoff Doppel- und Dreifachbindungen wie Acetylene, Acetylide, 1,2 Diene; gespannte Ringverbindungen wie Azirine oder Epoxide, Verbindungen mit aneinander grenzenden N-Atomen wie Azo- und Diazoverbindungen, Hydrazine, Azide, Verbindungen mit aneinander grenzenden O-Atomen wie Peroxiden und Ozoniden, Sauerstoff-Stickstoff-verbindungen wie Hydroxylamine, Nitrate, N-Oxide, 1,2 Oxalate, Nitro- und Nitroso-verbindungen; Halogen-Stickstoffverbindungen wie Chloramine und Fluoramine, Halogen-Sauerstoffverbindungen wie Chlorate, Perchlorate, Jodosyl-verbindungen; Schwefel-Sauerstoff-verbindungen wie Sulphonylhalide, Sulphonylcyanide, und Verbindungen mit Kohlenstoff-Metall-Bindungen und Stickstoff-Metall-Bindungen wie Grignard-reagenzien oder Organo-Lithium-verbindungen. Jedoch können auch viele andere organische Verbindungen ohne die genannten funktionellen Gruppen und eine Vielzahl anorganischer Verbindungen deflagrationsfähig sein.

Grundsätzlich gelten alle Stoffe mit einer Zersetzungsenthalpie von größer oder gleich 500 J/g als potentiell deflagrationsfähig. Es sind auch Stoffe mit Zersetzungsenthalpie von 300 - 500 J/g bekannt, welche deflagrationsfähig sind.

Die Deflagrationsfähigkeit einer Substanz muss im Einzelfall gesondert ermittelt werden.

Zur Prüfung des Deflagrationsverhaltens von Stoffen und Stoffgemischen sind verschiedene Prüfmethoden bekannt.

In dem UN Prüfbuch "Transportation of Dangerous Goods, Manual of Tests and Criteria", 5th Revised Edition, 2009, werden in Sektion 23 (S 237 ff) 2 Prüfmethoden zur Ermittlung der Deflagrationsfähigkeit beschrieben.

In dem Test C.1 ("Time/Pressure Test") werden 5 g der zu prüfenden Substanz in einem Druckgefäß mit ca. 17 ml Inhalt gezündet. Kriterien für die Bewertung sind die Erreichung eines Grenzdrucks von ca. 20,7 barü, sowie die Zeit nach Zündung, in welcher der Grenzdruck erreicht wird. (Barü = Bar Überdruck)

Die Deflagrationsfähigkeit wird im Test C.1 wie folgt beurteilt:
- Ja, schnell deflagrationsfähig, wenn der Druck innerhalb des Druckgefäßes nach Zündung in weniger von 30 Sekunden von 6,90 barü auf 20,70 barü ansteigt.
- Ja, langsam deflagrationsfähig, wenn der Druck innerhalb des Druckgefäßes nach Zündung in 30 Sekunden oder länger von 6,90 barü auf 20,70barü ansteigt.
- Nicht deflagrationsfähig, wenn der Grenzdruck von 20,70 barü nicht erreicht wird.

In dem Test C.2 wird eine Probe in ein Dewar-Gefäß mit etwa 48 mm innerem Durchmesser und einer Höhe von 180 - 200 mm gefüllt. Das Gemisch wird mit einer offenen Flamme gezündet.

Die Deflagrationsfähigkeit wird im Test C.2 wie folgt beurteilt:
- Ja, schnell deflagrationsfähig, wenn die Deflagrationsgeschwindigkeit größer 5 mm/sec ist.
- Ja, langsam deflagrationsfähig, wenn die Deflagrationsgeschwindigkeit zwischen 0,35 mm/sec bis 5 mm/sec liegt.
- Nicht deflagrationsfähig, wenn die Deflagrationsgeschwindigkeit kleiner 0,35 mm/sec ist, oder die Reaktion vor Erreichen der unteren Marke verlöscht.

Insgesamt wird eine Substanz als nicht deflagrationsfähig eingestuft, wenn die Substanz im Test C.1 nicht als "schnell deflagrationsfähig" und im Test C.2 als nicht deflagrationsfähig klassifiziert wurde.

Ein weiterer Test zur Ermittlung der Deflagrationsfähigkeit ist in VDI2263-1 (1990, S. 13 ff.) beschrieben.

In der Prüfung nach VDI2263-1 wird eine Substanz in ein unten geschlossenes Glasrohr mit ca. 5 cm Durchmesser gefüllt, in welchem in verschiedenen Höhen radial versetzt mehrere Thermoelemente angebracht sind. Nach örtlicher Initiierung durch eine Glühwendel, eine Glühkerze, einen Mikrobrenner oder eine Zündmischung aus Blei(IV)-Oxid und Silizium wird die Fortschreitung der Zersetzung ermittelt. Die Initiierung erfolgt von oben und vom Boden der Schüttung aus. Breitet sich die Zersetzung in wenigstens der Versuche (Zündung von oben und Zündung von unten) aus, so gilt der Stoff als deflagrationsfähig.

Als Zündquellen alternativ verwendet werden Glühwendel, Glühkerze, Mikrobrenner oder eine Zündmischung (Silizium/Bleioxid im Verhältnis 3:2). Die Einwirkdauer und der Energieeintrag der Zündquellen sind nicht weiter definiert.

In der Standard-Ausführung nach VDI2263-1 wird das Deflagrationsverhalten bei Umgebungstemperatur- und Druck gemessen. Es kann aber auch bei erhöhter Temperatur und im geschlossenen Gefäß gemessen werden.

Es ist bekannt, dass viele Stoffe sich in dem Test nach VDI2263-1 ohne die Ausbildung einer geschlossenen Front und auch nicht vollständig zersetzen. Häufig kommt es innerhalb der Schüttung zur Bildung von Kanälen, in deren Inneren die Zersetzung fortschreitet, während das umgebende Material sich nicht zersetzt. Ein solches Verhalten bedeutet aber für die Verarbeitung eines Stoffes ein Gefährdungspotential. Der Fachmann wird für die Prüfung des Deflagrationsverhaltens eines Stoffes oder Stoffgemisches die Parameter so auswählen, dass die Situation während der Verarbeitung am besten wiedergegeben wird. So wird eine Substanz für den Test nach VDI2263-1 auf die Temperatur gebracht werden, bei welcher auch die Verarbeitung der Substanz erfolgt. Bezüglich der Zündquelle kann man davon ausgehen, dass keine Deflagrationsfähigkeit vorliegt, wenn nach 300 Sekunden Einwirkzeit bei einer Temperatur > 600 °C, zum Beispiel durch eine Glühwendel oder eine Glühkerze, letztere bei einer Energieaufnahme von 40 W, entspricht, , noch keine Fortschreitung der Reaktion zu beobachten ist. Bei der Fortschreitung der Reaktion ist jede Art der Fortführung der Zersetzung, welche sich durch die Schüttung fortpflanzt, als Zeichen für ein Deflagrationsverhalten zu werten, auch wenn eine Kanalbildung vorliegt und die Schüttung nicht in voller Breite unter Ausbildung einer Zersetzungsfront durchreagiert.

In VDI-Bericht 975 (1992) Seite 99 ff wird eine Klassierung deflagrationsgefährlicher pulverförmiger Stoffe beschrieben. Die deflagrationsfähigen Stoffe werden in 3 Gefahrenklassen eingeteilt. Während Stoffe der Gefahrenklasse 3 grundsätzlich nicht in Apparaten mit mechanischen Einbauten verarbeitet werden dürfen, können Stoffe der Gefahrenklassen 1 und 2 unter gewissen Voraussetzungen in Apparaten mit mechanischen Einbauten verarbeitet werden.

Wichtige Kriterien für die Einstufung in eine der 3 Gefahrenklassen ist die Kerzeneinschaltzeit, das heißt die Zeit, welche die Zündquelle im Test VDI2263-1 vom Einschalten bis zum Sichtbarwerden der Zersetzungsreaktion eingeschaltet ist. Die Autoren vergleichen die Kerzeneinschaltzeit mit der Mindestzündenergie bei Staubexplosionen. Die Kerzeneinschaltzeit lässt sich im Hinblick auf die Verarbeitung in einem Produktionsapparat auch interpretieren als die Zeitspanne, mit welcher eine Zündquelle wie eine heiße Anlaufstelle oder eine heiße Schraube auf die sie umgebende Substanz einwirken kann, bevor durch Abkühlung der Anlaufstelle oder Schraube oder Erneuerung der Umgebung um die heiße Stelle wieder ein unkritischer Zustand erreicht wird. Es gilt somit: "Je kürzer die Kerzeneinschaltzeit, umso leichter ist die Deflagration auszulösen". Die Autoren nennen eine Kerzeneinschaltzeit von ≤ 20 sec als Grenzwert für eine Einordnung in die Gefahrenklasse 3, und einen Grenzwert von > 60 sec als Grenzwert für eine Einordnung in die Gefahrenklasse 1.

Die Herstellung von deflagrationsfähigen Feststoffen erfolgt mit den üblichen, aus der organischen und anorganischen Chemie bekannten Verfahrensschritten. Meist werden Einsatzstoffe in flüssiger Form oder in Form von Lösungen miteinander zur Reaktion gebracht, der gewünschte Stoff fällt üblicherweise als Feststoff aus. Dieser wird dann von den verbliebenen flüssigen Komponenten abgetrennt, und steht nach eventuell weiteren Reinigungsschritten, Trocknung und Zwischenlagerung in gewünschter Form für die Abfüllung und den Transport zu den Abnehmern zu Verfügung. Gegebenenfalls wird der gewünschte Stoff weiter verarbeitet und zum Beispiel gemahlen und/oder mit anderen Komponenten vermischt.

Die Herstellung von deflagrationsfähigen Feststoffen im Labormaßstab ist in der Regel unproblematisch. Die gehandhabten Mengen sind klein, die Wahrscheinlichkeit der Initiierung einer Deflagration ist gering, eventuell auftretende Deflagrationen werden schnell erkannt und selbst bei Nichterkennen und Fortschreiten einer Deflagration ist das Schadensausmaß gering.

Problematisch ist jedoch die Herstellung deflagrationsfähiger Stoffe in größeren Mengen wie sie in einem Technikumsbetrieb oder in einem Produktionsbetrieb erfolgt. Hierbei kommen eine Reihe von Apparaten zum Einsatz, welche einerseits potentielle Initiierungsquellen aufweisen, und bei welchen andererseits eine Deflagration unter Umständen erst eine längere Zeit nach der Initiierung detektiert werden kann.

Apparate in Technika und Produktionsbetrieben sind häufig mit mechanischen Einrichtungen bestückt, welche dem Transport, der Durchmischung, der Erneuerung der Oberfläche oder andern Zwecken dienen.

So werden zum Beispiel für die Homogenisierung von Feststoffen Mischer mit bewegten mechanischen Elementen wie zum Beispiel Pflugscharmischer oder Schneckenmischer eingesetzt. Es ist bekannt, dass die mechanischen Einrichtungen eine der häufigsten Ursachen für die Initiierung einer Deflagration sind. So kann bei einer Störung ein bewegtes Mischelement in direkten Kontakt mit dem Apparatemantel kommen, an der Reibungsstelle erfolgt eine lokalen Erhitzung, welche den umgebenden Stoff zur Zersetzung bringen und so eine Deflagration initiieren kann. Es sind ebenfalls Fälle bekannt, bei welchen ein Fremdkörper, zum Beispiel eine Schraube, in einen Apparat gelangt war, dort zwischen Wandung und Rühr-/Mischorgan gelangte und durch die Erhitzung eine Deflagration auslöste. Selbst durch Reiben harter Krusten oder durch Reibung in einer verstopften Förderschnecke ist es zur Auslösung von Deflagrationen gekommen. Auch ist bekannt, dass Deflagrationen von einem Apparat in den anderen übertragen werden können. So kann in einem Mischer eine eingeschleppte Schraube in der beschriebenen Weise durch Reibung erhitzt werden. Die heiße Schraube wird dann zum Beispiel in ein Silo ohne mechanische Einbauten ausgetragen. Die Temperatur der Schraube kann noch hinreichend hoch sein, um in dem Silo die umgebende Substanz zur Zersetzung zu bringen und so eine Deflagration auszulösen. In gleicher Weise können Agglomerate, in welchen bereits eine deflagrative Zersetzung ausgelöst wurde, in einen Apparat ohne mechanische Einbauten ausgetragen werden und dort die deflagrative Zersetzung des Apparateinhalts initiieren.

Es ist eine Reihe von Maßnahmen bekannt, welche eine sichere Verarbeitung deflagrationsfähiger Stoffe ermöglichen.

Im VDI-Bericht 975 (1992) Seite 99 ff wird eine Systematik dargelegt, wie bei der Beurteilung und Auswahl von Maßnahmen bei der Verarbeitung von deflagrationsgefährlicher pulverförmigen Stoffen vorzugehen ist In dem Bericht wird eine Klassierung der deflagrationsfähigen Stoffe in 3 Gefahrenklassen beschrieben, wobei Stoffe in der Gefahrenklasse 3 das höchste Gefährdungspotential aufweisen und Stoffe in der Gefahrenklasse 1 das niedrigste Gefährdungspotential. Entsprechend der Gefährdungsklasse werden geeignete Verarbeitungsverfahren aufgeführt. Obwohl die in der genannten Veröffentlichung genannten Kriterien keine allgemeine Gültigkeit haben, stellt die in dieser Veröffentlichung dargelegte Systematik einen guten Ansatz dar, wie bei der Beurteilung und Verarbeitung von deflagrationsfähigen Stoffen vorzugehen ist. Weitere Beispiele für eine sichere Verarbeitung deflagrationsfähiger Stoffe finden sich auch in dem VDI-Bericht 1272 (1996), Seite 441 ff. Bei Stoffen mit einer hohen Deflagrationsneigung wird darauf geachtet, dass die Verarbeitung ohne mechanische Einwirkungen erfolgt. Dies geschieht beispielsweise dadurch, dass eine Trocknung auf einzelnen Tabletts in einem Trockenschrank erfolgt, statt in einem Trockner mit mechanischen Einbauten wie zum Beispiel einem Schaufeltrockner. Die Verarbeitung ohne mechanische Einrichtungen ist jedoch sehr aufwendig. Vielfach muss der Stofftransport manuell erfolgen, was neben dem hohen Aufwand auch zur Gesundheitsgefährdung des Bedienpersonals und zu Qualitätsproblemen führen kann. Die Verarbeitung ohne mechanische Einrichtung und wird nur dann in Betracht kommen, wenn eine sichere Verarbeitung mit mechanischen Einrichtungen nicht möglich ist. In der zuvor zitierten Veröffentlichung In dem VDI-Bericht 975 (1992) Seite 99 ff sind zum Beispiel bei den Stoffen der Gefährdungsklasse 3 nur Verarbeitungsverfahren ohne mechanische Einrichtung vorgesehen.

Für Stoffe, bei welchen das Gefährdungspotential durch Deflagration weniger ausgeprägt ist, können unter bestimmten Bedingungen auch mit mechanischen Einrichtungen verarbeitet werden. In der zitierten Veröffentlichung In den VDI-Bericht 975 (1992) Seite 99 ff gilt dies für Stoffe der Gefahrenklassen 1 und 2.

Ein gängiges Verfahren zur Vermeidung von Deflagrationen ist die sorgfältige Vermeidung des Eintrags von Fremdkörpern. Dies kann zum Beispiel durch eine Metallabscheidung erfolgen, welche vor den Eintrag in den Apparat erfolgt und das Mitschleppen von Schrauben und anderen metallischen Fremdkörpern in den Verarbeitungsschritt verhindert.

Auch kann beim Bau der Apparate auf die Vermeidung möglicher Zündquellen geachtet werden, zum Beispiel, in dem die Abstände zwischen mechanischem Mischer und Wandung groß gewählt werden.

Die genannten Verfahren der Zündquellenvermeidung können das Risiko einer Deflagration deutlich mindern, ausschließen lässt sich die Deflagration hierdurch aber nicht. Die genannten Methoden sind zudem aufwändig und in manchen Fällen mit einer Beeinträchtigung der Leistung der Apparate verbunden.

Eine weitere Möglichkeit zur Vermeidung einer Deflagration besteht darin, die deflagrationsfähige Substanz mit einer weiteren, nicht deflagrationsfähigen und nicht katalytisch wirkenden Substanz zu vermischen. Nachteilig bei dieser Maßnahme ist, dass die gewünschte Substanz nicht in der gewünschten Zusammensetzung erhalten werden kann. Die Reduzierung der Deflagrationsfähigkeit durch Zugabe einer weiteren Substanz ist zum Beispiel in US5268177 beschrieben.

Ein weiteres Verfahren zur sicheren Verarbeitung von deflagrationsfähigen Substanzen besteht darin, den bei einer Deflagration entstehenden Druck bzw. die bei der Deflagration entstehenden Gase sicher abzuführen. Dies kann zum Beispiel durch Einbau entsprechend dimensionierter Berstscheiben und entsprechender Ableiteinrichtungen erfolgen. Hierbei ist zu beachten, dass die Deflagrationsgeschwindigkeit mit steigendem Druck zunimmt, Ansprechdruck und Ableitung sind entsprechend auszulegen. Auch ist darauf zu achten, dass mitgerissene Substanzen an einer Fortführung der Deflagration gehindert werden müssen. Dies kann zum Beispiel durch Einleitung der abgeführten Gase in ein Wasserbad erfolgen.

Ein weiteres bekanntes Verfahren zur sicheren Verarbeitung von deflagrationsfähigen Substanzen besteht darin, den Beginn einer Deflagration rechtzeitig zu erkennen und die beginnende Deflagration durch Abführen der Energie zu unterbinden. Die Erkennung kann über eine Reihe von Indikatoren erfolgen. Bekannt sind zum Beispiel die Überwachung von Temperatur oder/und Druck. Es kann aber auch das Auftreten gewisser Zersetzungsgase wie Kohlenmonoxid detektiert werden. Bei Erreichen des Auslösewertes wird die Energie aus dem System abgeführt. In der Regel erfolgt dies durch Zugabe einer größeren Menge an Wasser. Durch die Wärmekapazität des Wassers kühlt sich die deflagrierende Substanz auf Temperaturen unterhalb der Zersetzungstemperatur ab. Eine zusätzliche Wärmeabfuhr kann durch die Entstehung von Wasserdampf erfolgen. Dem Wasser kann ein Detergenz zugegeben werden, um eine gute Benetzung der deflagrierenden Substanz zu gewährleisten.

Nachteilig an den vorgenannten Verfahren ist, dass sie nur Schadensbegrenzend wirken und erst nach Auslösung der Deflagration wirksam werden. Somit führen diese Verfahren zum Verlust zumindest eines Teiles der Substanz führen, da diese sich zum Teil zersetzt und die unzersetzten Teile durch Wasser und andere Reagenzien in der Regel unbrauchbar gemacht werden. Problematisch ist auch die sichere Entfernung von entstehendem Wasserdampf.

Es bleibt festzustellen, dass die bisher beschriebenen Verfahren zur Verarbeitung deflagrationsfähiger Substanzen Nachteile aufweisen.

Die Aufgabe der vorliegenden Erfindung lag daher darin, bessere Maßnahmen für die Verarbeitung und/oder Handhabung deflagrationsfähiger Feststoffe oder Feststoffgemische bereitzustellen. Insbesondere sollten diese Maßnahmen die Wahrscheinlichkeit der Auslösung einer Deflagration verringern, ohne die Stoffeigenschaften durch Zugabe eines weiteren Stoffes zu verändern.

Die Aufgabe wird durch ein Verfahren gelöst, wobei die Verarbeitung und / oder Handhabung der deflagrationsfähigen Feststoffe in einer Umgebung unter reduziertem Druck erfolgt. Es wurde überraschenderweise gefunden, dass die Auslösung einer Deflagration bei der Verarbeitung und Handhabung deflagrationsfähiger Stoffe in einer Umgebung unter reduziertem Druck deutlich verzögert werden kann.

Eine Verzögerung der Auslösung einer Deflagration lässt sich überraschenderweise schon durch eine leichte Verminderung des Drucks innerhalb des Apparates unterhalb des Umgebungsdrucks/atmosphärischen Drucks erreichen. So wurde schon bei einer Reduktion des Drucks innerhalb des Gefäßes auf unter oder gleich 800 mbara eine deutliche Verzögerung festgestellt (bara = bar absolut). Bevorzugt erfolgt die Verarbeitung und Handhabung bei möglichst niedrigem Druck innerhalb des Apparates. Bevorzugt für die Verarbeitung ist ein Druckbereich von ≤ 500 mbara, besonders bevorzugt ein Druckbereich ≤ 100 mbara, besonders bevorzugt in ein Druckbereich ≤ 20 mbara. Aus wirtschaftlichen und technischen Gründen wird als untere Grenze des Druckbereichs innerhalb des Gefäßes ≥2 mbara, bevorzugt ≥10 mbara empfohlen.

Das erfindungsgemäße Verfahren ist für die Verarbeitung und Handhabung deflagrationsfähiger fester Substanzen einschließlich explosionsfähiger fester Substanzen anwendbar.

Als deflagrationsfähig im Sinne dieser Erfindung gelten alle Stoffe, welche entweder nach dem UN Prüfbuch "Transportation of Dangerous Goods, Manual of Tests and Criteria", 5th Revised Edition, 2009, Deflagration, unter Abschnitt 23.2.2 genannten Kriterien als deflagrationsfähig einzuordnen sind (Question "Can it propagate adeflagration?" - Answer "yes, rapidely" oder" yes, slowly"), und / oder im Test VDI2263-1 bei Ausprüfung bei der in der Verarbeitung vorgesehenen Temperatur und einer Zündung von oben oder unten mit einer Zündpille, Zündwendel oder Glühkerze, letztere mit einer aufgenommenen Leistung von mindestens 40 W und einer Einwirkdauer von 300 Sekunden eine sich selbsttätige Zersetzung aufweisen, wobei die Zersetzung ein Form einer Zersetzungsfront als auch in Form von Zersetzungskanälen fortschreiten kann.

Typische deflagrationsfähigen Stoffe im Sinne der vorliegenden Erfindungen sind organische Verbindungen mit funktionellen Gruppen wie Kohlenstoff-Kohlenstoff Doppel- und Dreifachbindungen wie Acetylene, Acetylide, 1,2 Diene; gespannte Ringverbindungen wie Azirine oder Epoxide, Verbindungen mit aneinander grenzenden N-Atomen wie Azo- und Diazoverbindungen, Hydrazine, Azide, Verbindungen mit aneinander grenzenden O-Atomen wie Peroxiden und Ozoniden, Sauerstoff-Stickstoff-verbindungen wie Hydroxylamine, Nitrate, N-Oxide, 1,2 Oxalate, Nitro- und Nitroso-verbindungen; Halogen-Stickstoffverbindungen wie Chloramine und Fluoramine, Halogen-Sauerstoffverbindungen wie Chlorate, Perchlorate, Jodosyl-verbindungen; Schwefel-Sauerstoff-verbindungen wie Sulphonylhalide, Sulphonylcyanide, und Verbindungen mit Kohlenstoff-Metall-Bindungen und Stickstoff-Metall-Bindungen wie Grignard-reagenzien oder Organo-Lithium-verbindungen. Deflagrationsfähigen Feststoffe sind deflagrationsfähigen Stoffe in fester Form, wobei der Stoff rein oder gemischt in fester Form ist, z.B. als Pulver oder Granulate in beliebiger Korngröße vorhanden ist. Als deflagrationsfähige Feststoffe im Sinne dieser Erfindung gelten auch deflagrationsfähige Flüssigstoffe, welche an nicht deflagrationsfähigen Feststoffen resorbiert sind und somit in fester Form vorliegen. Als deflagrationsfähige Feststoffe im Sinne dieser Erfindung gelten ebenfalls deflagrationsfähige Stoffe in fester Form, welche noch Reste von Wasser oder andere Flüssigkeiten wie Lösungsmittel aufweisen (feuchte Feststoffe). Die Korngröße und die Korngrößenverteilung haben bekanntermaßen Einfluss auf das Deflagrationsverhalten, die beiden Parameter stellen für die vorliegende Erfindung aber keine Einschränkung dar.

Bei den durchgeführten Experimenten (Siehe Beispiele 1 bis 4) nach VDI VDI2263-1 verlängerten sich die Zündzeiten oder Kerzeneinschaltzeiten durch Anlegen eines reduzierten Drucks innerhalb des Messgefäßes um einen Faktor 2 bis 8. Entsprechen den in dem VDI-Bericht 975 (1992) Seite 99 ff genannten Kriterien sinkt bei Verlängerung der Zündzeiten oder Kerzeneinschaltzeiten die Wahrscheinlichkeit, dass eine Deflagration ausgelöst werden kann. Unter reduziertem Druck verhalten sich deflagrationsfähige Feststoffe nach den o. g. Kategorisierungen weniger deflagrationsfähig, was wiederum die Anwendung insbesondere von Apparate mit mechanischen Einbauten unter einem minderen Deflagrationsrisiko ermöglicht.

Verarbeitung und Handhabung im Sinne dieser Anmeldung sind Verfahrens- und Handhabungsschritte zur Herstellung, Verarbeitung, Lagerung und dem Transport von deflagrationsfähigen Feststoffen, insbesondere Filtrieren, Trocknen, Mahlen, Sieben, Mischen, Homogenisieren, Granulieren, Kompaktieren, Abfüllen, Lagern und Transport in einem Transportbehälter, sowie mechanische Transport wie z.B. die die Förderung in Förderschnecken oder durch Zellradschleusen. Im Sinne der Erfindung können diese Verfahrensschritte sowohl in oder mit Hilfe von Apparaten, bei welchen der bearbeitete Feststoff mit Hilfe mechanischer Einrichtungen bewegt wird, wie zum Beispiel in einem Pflugscharmischer, durchgeführt werden, als auch in oder mit Hilfe von Apparaten ohne mechanische Einrichtungen, wie zum Beispiel Silos. Besonders vorteilhaft ist das Verfahren für Verarbeitung und Handhabung deflagrationsfähiger Feststoffe in Apparate mit mechanischen Einbauten. Aus dem Stand der Technik sind Verarbeitung Lagern und Transport in oder mit Hilfe von Apparaten ohne mechanische Einbauten unter reduziertem Druck zur Minderung des Explosionsrisikos von explosionsfähigen Feststoffen oder zum Schutz vor Schädigung durch Luftsauerstoff bekannt. Der reduzierte Druck steht aber im Zusammenhang mit der Bereitstellung einer inerten Atmosphäre.

Auch das Trocknen unter reduziertem Druck ist allgemein bekannt. Aber hier beschleunigt der reduzierte Druck das Trocknen und wird nicht mit einer Minderung des Deflagrations- und Explosionsrisikos von deflagrations- und explosionsfähigen Feststoffe in Verbindung gebracht.

Die überraschende Minderung des Deflagrations- und Explosionsrisikos von deflagrations- und explosionsfähigen Feststoffe geschieht im Vergleich zum Stand der Technik zur Handlung von explosionsfähigen Stoffgemischen unabhängig davon, ob die Verarbeitung und /oder Handhabung unter inerter Atmosphäre durchgeführt wird.

Gegenstand der Erfindung ist daher ein Verfahren zur Verarbeitung und / oder Handhabung deflagrationsfähiger Feststoffe mit einem oder mehreren Verfahrensschritten aus der Gruppe umfassend Filtration, Mahlen, Sieben, Mischen, Homogenisieren, Granulieren, Kompaktieren, Abfüllen, Trocknen, Lagern und Transport in einem Transportbehälter sowie sonstige Schritten in Apparate mit mechanischen Einbauten, **dadurch gekennzeichnet, dass** die Verarbeitung und / oder Handhabung in einer Umgebung unter reduziertem Druck erfolgt.

Die Reduzierung des Drucks in den Apparaten erfolgt durch dem Fachmann bekannte Techniken mittels Vakuumpumpen wie zum Beispiel Verdrängerpumpen, Strahlpumpen, Drehschieberpumpen, Zentrifugalpumpen, Wasserringpumpen, Wälzkolbenpumpen und anderen zur Erzeugung des gewünschten Drucks geeignete Aggregate.

Bei der Herstellung deflagrationsfähiger Stoffe werden häufig Mischer mit mechanischen Einbauten wie zum Beispiel Pflugscharmischer oder Schnecken-Mischer ("Nauta-Mischer") zur Homogenisierung oder Untermischen von Additiven eingesetzt. Die Mischer werden in der Regel bei Normaldruck betrieben. Teilweise sind in solchen Mischern zusätzlich Zerkleinerungswerkzeuge ("Zerhacker) eingebaut. Durch Fehlfunktion, zum Beispiel durch eine Deformation des Mischorgans oder durch den Eintrag einer Schraube, kann es durch Reibung zu einer lokalen Erhitzung kommen, durch welche eine Deflagration ausgelöst werden kann. Wird ein solcher Mischer statt bei atmosphärischen Druck in einem Apparat unter reduziertem Druck betrieben, so kann die Wahrscheinlichkeit der Auslösung einer Deflagration stark vermindert werden, das Risiko eine unkontrollierten Zersetzung des Apparateinhalts sinkt, und die Sicherheit der Anlage wird deutlich erhöht.

Als weitere Anwendung für die Verbesserung durch die erfindungsgemäße Maßnahme wird die Filtration in einer Filternutsche beschrieben. In einer Filternutsche wird in der Regel eine Suspension auf ein Sieb oder sonstiges Filtermedium aufgetragen. Das Filtrat dringt aufgrund der Schwerkraft durch das Sieb oder Filtermedium, die Filtrationsgeschwindigkeit kann durch Unterduck auf der Filtratseite und/oder Überdruck auf der Zugabeseite erhöht werden. Zur Verstetigung der Filtration und des Filterkuchens wird die Suspension in der Regel mittels eines Rührers gerührt. Solange sich auf der Zugabeseite Flüssigkeit befindet, ist die Gefahr einer Deflagration gering. Nach Abtrennen der flüssigen Phase steigt das Risiko einer Deflagration. Durch mechanische Einbauten, zum Beispiel den Rührer, kann es bei Fehlfunktion durch Reibungshitze zum Auslösen einer Deflagration kommen. Erfindungsgemäß wird der Filterkuchen unter reduziertem Druck gehalten. Dieses kann zum Beispiel durch Anlegen eines leichten Unterdrucks auf der Zugabeseite von zum Beispiel 500 mbara bei stärkerem Unterdruck von zum Beispiel 20 mbara auf der Filtratseite erfolgen, wobei noch eine Druckdifferenz über dem Filter aufrechterhalten bleibt. Ebenso kann erfindungsgemäß zum Ende oder nach Beendigung der Filtration und vor Einschalten der mechanischen Einrichtungen wie Rührer der Apparat auf der Aufgabeseite oder auch der gesamte Apparat auf einen erfindungsgemäßen Druck unterhalb des Atmosphärendrucks gebracht werden. Es kann auch dergestalt vorgegangen werden, dass während der Anwesenheit der flüssigen Phase auf dem Filter der Rührer eingeschaltet ist, bei Absinken des Flüssigkeitsstandes zur Vermeidung der Auslösung einer Deflagration der Rührer ausgeschaltet wird und erst nach Erzeugung eines erfindungsgemäßen Unterdrucks wieder eingeschaltet wird.

Der Austrag aus einer Filternutsche erfolgt in der Regel durch ein mechanisches Austragsorgan. Dies kann zum Beispiel durch den Rührer erfolgen, welcher zum Austrag in umgekehrter Drehrichtung gefahren wird, oder eine separate mechanische Austragsvorrichtung. Bei Fehlfunktion kann es durch Reibungshitze zum Auslösen einer Deflagration kommen. Erfindungsgemäß erfolgt der Austrag aus einer Filternutsche bei einem Druck unterhalb des Atmosphärendrucks, wodurch die Wahrscheinlichkeit eines Eintretens einer Deflagration deutlich reduziert wird.

Als weitere Anwendungen für die Verbesserung durch die erfindungsgemäße Maßnahme werden der Transport deflagrationsfähiger Stoffe mittels Förderschnecken oder Zellradschleusen beschrieben.

Der Transport von Feststoffen erfolgt häufig durch Förderschnecken, welche in einem Rohr oder rohrähnlichen Apparat eingebaut sind. Durch Reibung der Schnecke an der Wandung, oder durch Eintrag eines Fremdkörpers wie einer Schraube in die Schnecke kann es durch Reibungshitze zum Auslösen einer Deflagration kommen. Es sind auch Fälle bekannt, bei welchen schon durch die Kompression in einer verstopften Förderschnecke Deflagrationen ausgelöst wurden. Erfindungsgemäß wird der Druck in dem die Förderschnecke umgebenden Apparat auf einem Druck unterhalb des Atmosphärendrucks reduziert, wodurch die Wahrscheinlichkeit eines Eintretens einer Deflagration deutlich reduziert wird.

Zellradschleusen werden häufig beim Übergang von einem Apparat zu einem anderen Apparat eingesetzt. Durch Reibung des Zellrades an der Wandung, oder durch Eintrag eines Fremdkörpers wie einer Schraube in die Zellradschleuse kann es durch Reibungshitze zum Auslösen einer Deflagration kommen. Erfindungsgemäß wird der Druck in der Zellradschleuse auf einem Druck unterhalb des Atmosphärendrucks reduziert, wodurch die Wahrscheinlichkeit eines Eintretens einer Deflagration deutlich reduziert wird.

Durch die vorgenannten Förderschnecken oder Zellradschleusen, oder auch durch andere Fördertechniken gelangen deflagrationsfähige Stoffe in Apparate ohne mechanische Einbauten wie zum Beispiel Pufferbehälter, Silobehälter, Transportbehälter oder andere Behälter.

Durch mit eingetragene heiße Fremdkörper, wie zum Beispiel eine durch Reibung in einer Förderschnecke erhitzte Schraube, kann auch in Apparaten ohne mechanische Einrichtungen eine Deflagration ausgelöst werden. Erfindungsgemäß werden diese Apparate während und nach der Befüllung bei einem Druck unterhalb des Atmosphärendrucks gehalten, wodurch die Wahrscheinlichkeit eines Eintretens einer Deflagration deutlich reduziert wird.

Ein besonderes Problem bei der Verarbeitung deflagrationsfähiger Stoffe stellt die Zerkleinerung und das Mahlen dar. In Mühlen, Brechern und analogen Zerkleinerungsorganen wird mechanische Energie in das Mahlgut eingebracht, es kommt schon im bestimmungsgemäßen Betrieb zu einer Erwärmung durch Reibung, durch welche eine Deflagration ausgelöst werden kann. Bei Eintrag eines Fremdkörpers wie zum Beispiel einer Schraube steigt die Wahrscheinlichkeit für die Auslösung einer Deflagration deutlich an. Erfindungsgemäß wird die Mühle bzw. das Zerkleinerungsorgan bei einem Druck unterhalb des Atmosphärendrucks betrieben, wodurch die Wahrscheinlichkeit eines Eintretens einer Deflagration deutlich reduziert wird. Bei den Mühlen oder Zerkleinerungsorganen kann es sich um bekannte Mühlen wie zum Beispiel Walzenbrecher, Stachel- oder Zahnwalzenbrecher handeln.

Auch in Sieben und Reibsieben oder Passiersieben wie zum Beispiel Frewitt-Siebe kann es bei Fehlfunktionen zu Reibungswärme und in der Folge zu der Auslösung einer Deflagration kommen. Erfindungsgemäß erfolgt das Sieben bzw. das Sieben mit einem Reibsieb oder Passiersieb bei einem Druck unterhalb des Atmosphärendrucks, wodurch die Wahrscheinlichkeit eines Eintretens einer Deflagration deutlich reduziert wird.

Bei der Trocknung von Feststoffen werden diese in der Regel durch mechanische Einbauten bewegt, um die Oberfläche ständig zu erneuern und so den Stoff- und Wärmetransport zu verbessern. Typische Trockner sind zum Beispiel Schaufeltrockner oder Tellertrockner. Auch sind bestimmte der zuvor beschriebenen Filternutschen so ausgestattet, dass in diesen Apparaten nach der Filtration ein Trocknungsschritt nachgeschaltet werden kann. Durch Fehlfunktion, zum Beispiel durch eine Deformation des Mischorgans oder durch den Eintrag einer Schraube, kann es durch Reibung zu einer lokalen Erhitzung kommen, durch welche eine Deflagration ausgelöst werden kann.

Die Trocknung kann auch in Apparaten ohne mechanische Einbauten, wie zum Beispiel in einem Wirbelschichttrockner erfolgen. Auch in solchen Aggregaten kann es durch Eintrag von Fremdkörpern unter ungünstigen Umständen zu einer Deflagration kommen, zum Beispiel durch Fehlfunktion eines mechanischen Rechens im Eintrittsbereich.

Die Trocknung erfolgt in der Regel in der Weise, dass ein heißes Gas wie zum Beispiel heiße Luft oder heißer Stickstoff durch den Trockner geleitet werden (= mittels Gaskonvektionströmungen). Die heißen Gase bewirken sowohl den Energieeintrag zur Verdampfung als auch den Stofftransport. Der Energieeintrag kann auch durch Beheizen der Wandung oder beheizte Einbauten erfolgen. Statt im Gasstrom kann die Trocknung auch im Vakuum erfolgen. Der Einfluss eines reduzierten Drucks auf die Deflagrationsneigung war bisher nicht bekannt/untersucht worden, so dass für die Entscheidung für eine Trocknung unter Vakuum andere Kriterien wie der Siedepunkt des Lösemittels oder der Schmelzpunkt der zu trocknenden Substanz zu Grunde gelegt wurden. Erfindungsgemäß erfolgt die Trocknung deflagrationsfähiger Stoffe stets bei reduziertem Druck. Die Einstellung des reduzierten Drucks kann alleine durch Erzeugung des Unterdrucks mittels einer Pumpe, als auch durch Erzeugung des Unterdrucks mittels einer Pumpe und gleichzeitiger Zufuhr einer begrenzten Menge Gas in den Trockner zur Verbesserung des Stofftransports erfolgen. Bei beiden Maßnahmen wird die Wahrscheinlichkeit des Eintretens einer Deflagration deutlich reduziert.

Analog zu den beschriebenen Anwendungen ist zu erwarten, dass die Sicherheit auch in anderen Apparaten mit mechanischen Einbauten, sofern diese erfindungsgemäß unter reduziertem Druck betrieben werden, deutlich gesteigert werden kann.

### Beispiele

Folgende Experimente belegen den Einfluss des reduzierten Drucks auf die Deflagrationsfähigkeit von Azodicarbonamid ohne sich darauf zu begrenzen.

Es wurden Messungen zum Deflagrationsverhalten entsprechend VDI 2263 durchgeführt.

Die Messungen erfolgten in einem Metallrohr von 4,8 cm Durchmesser und 13,5 cm Höhe. Als Zündquelle diente eine Boden des Metallrohrs (= Prüfrohr) eingelassene Glühkerze des Typs 0 250 201 032-4FS der Firma Bosch. Das Prüfrohr wurde jeweils mit Azodicarbonamid 97% bezogen von der Firma Sigma-Aldrich gefüllt. Anschließend wurden vier 1,5 mm NiCr-Ni-Mantel-Thermoelemente mittig in der Weise in die Schüttung eingeführt, dass das erste Element sich 1 cm über der Spitze der Glühkerze und die anderen Elemente jeweils 2 cm höher befanden.

Für die Messungen wurde das Prüfrohr in einen Autoklaven von 4 1 innerem Volumen und einer inneren Höhe von 15,5 cm überführt. Das Prüfrohr wurde hierzu an einer am Autoklavendeckel fixierten Stange in der Weise befestigt, dass das Prüfrohr keinen Kontakt zum Mantel des Autoklaven hatte. Autoklav und Probe befanden sich auf Raumtemperatur.

Im Autoklavendeckel befanden sich gasdichte Durchlässe für die Drähte zur Beheizung der Zündquelle und für die Thermoelemente und eine Kapillare für einen außerhalb des Autoklaven angebrachten Druckaufnehmer sowie ein Ventil zum Evakuieren bzw. Entspannen des Apparates.

Eine Messung beginnt mit dem zeitgleichen Anlegen einer elektrischen Leistung und dem Starten der Temperatur-Zeit-Aufzeichnung. Die eingebrachte Leistung wurde über die Dauer der Messung konstant auf 40 W geregelt. Als Zeitpunkt für die Zündung des Stoffes wurde der Temperaturanstieg an der 1. Messstelle (1 cm über der Zündquelle) gewertet. Die Temperatur an der 1. Messstelle blieb nach Beginn des Anlegens der elektrischen Leistung nahezu konstant bzw. stieg langsam um einige °C an, bei Eintritt der Deflagration war ein starker Temperaturanstieg von ≥ 5°C/sec zu beobachten.

Der Anstieg der Temperaturen an den anderen Temperaturfühlern und der Druck im Autoklaven stiegen nach Beginn der Zündung jeweils zeitversetzt an.

### Beispiel 1 - Unter atmosphärischen Druck

Das oben beschriebene Prüfrohr wurde mit 85 g Azodicarbonamid (ADCA) gefüllt. Das Prüfrohr wurde in den Autoklaven überführt. Die Mischung wurde mittels der Glühkerze mit einer über die Dauer der Messung eingebrachten Leistung von 40 W erhitzt. Nach 19 Sekunden stieg die Temperatur an dem 1 cm oberhalb der Glühkerze angebrachten Temperaturfühler an.

Der Versuch wurde 2 Mal unter den gleichen Bedingungen wiederholt. Die Temperatur stieg jeweils nach 19 bzw. 15 Sekunden an.

Hiermit gehört ADCA gemäß VDI-Bericht 975 (1992) Seite 99 ff der Gefahrenklasse 3. (Nicht geeignet für Apparate mit mechanischen Einbauten)

### Beispiel 2 - Reduzierten Druck von 750 mbara

Das oben beschriebene Prüfrohr wurde mit 85 g Azodicarbonamid (ADCA) gefüllt. Das Prüfrohr wurde in den Autoklaven überführt, der Autoklav wurde auf 750 mbara mit Hilfe einer Pumpe evakuiert. Die Mischung wurde mittels der Glühkerze mit einer über die Dauer der Messung eingebrachten Leistung von 40 W erhitzt. Nach 34 Sekunden stieg die Temperatur an dem 1 cm oberhalb der Glühkerze angebrachten Temperaturfühler an.

Der Versuch wurde 2 Mal unter den gleichen Bedingungen wiederholt. Die Temperatur stieg jeweils nach 37 bzw. 41 Sekunden an.

### Beispiel 3 - Reduzierten Druck von 500 mbara

Das oben beschriebene Prüfrohr wurde mit 85 g Azodicarbonamid (ADCA) gefüllt. Das Prüfrohr wurde in den Autoklaven überführt, der Autoklav wurde auf 500 mbara mit Hilfe einer Pumpe evakuiert. Die Mischung wurde mittels der Glühkerze mit einer über die Dauer der Messung eingebrachten Leistung von 40 W erhitzt. Nach 53 Sekunden stieg die Temperatur an dem 1 cm oberhalb der Glühkerze angebrachten Temperaturfühler an.

Der Versuch wurde 2 Mal unter den gleichen Bedingungen wiederholt. Die Temperatur stieg jeweils nach 67 bzw. 65 Sekunden an.

### Beispiel 4 - Reduzierten Druck von 100 mbara

Das oben beschriebene Prüfrohr wurde mit 85 g Azodicarbonamid (ADCA) gefüllt. Das Prüfrohr wurde in den Autoklaven überführt, der Autoklav wurde auf 100 mbara mit Hilfe einer Pumpe evakuiert. Die Mischung wurde mittels der Glühkerze mit einer über die Dauer der Messung eingebrachten Leistung von 40 W erhitzt. Nach 149 Sekunden stieg die Temperatur an dem 1 cm oberhalb der Glühkerze angebrachten Temperaturfühler an.

Der Versuch wurde 2 Mal unter den gleichen Bedingungen wiederholt. Die Temperatur stieg jeweils nach 137 bzw. 189 Sekunden an.

Unter dem angewandten Unterdruck verhält sich ADCA wie ein deflagrationsfähigen Stoff der Gefahrenklasse 1 nach der Kategorisierung des VDI-Bericht 975 (1992) Seite 99 ff. (Verarbeitung in Apparate mit mechanischen Einbauten möglich).

### Beispiel 5 - Reduzierten Druck von 10 mbara

Das oben beschriebene Prüfrohr wurde mit 85 g Azodicarbonamid (ADCA) gefüllt. Das Prüfrohr wurde in den Autoklaven überführt, der Autoklav wurde auf 10 mbara mit Hilfe einer Pumpe evakuiert. Die Mischung wurde mittels der Glühkerze mit einer über die Dauer der Messung eingebrachten Leistung von 40 W erhitzt. Nach 172 Sekunden stieg die Temperatur an dem 1 cm oberhalb der Glühkerze angebrachten Temperaturfühler an.

Der Versuch wurde 2 Mal unter den gleichen Bedingungen wiederholt. Die Temperatur stieg jeweils nach 166 bzw. 190 Sekunden an.

## Patentansprüche

1. Verfahren zur Verarbeitung und / oder Handhabung von deflagrationsfähigen Feststoffen und Gemischen, **dadurch gekennzeichnet, dass** die Verarbeitung und / oder Handhabung in einer Umgebung unter reduziertem Druck erfolgt.

2. Verfahren nach Anspruch 1, **dadurch gekennzeichnet, dass** die Verarbeitung und / oder Handhabung in einer Umgebung mit einem Druck von ≤500 mbara erfolgt.

3. Verfahren nach einem der Ansprüche 1-2, mit einem oder mehreren Verfahrensschritten aus der Gruppe umfassend Filtration, Mahlen, Sieben, Mischen, Homogenisieren, Granulieren, Kompaktieren, Abfüllen, Trocknen, Lagern und Transport in einem Transportbehälter sowie sonstige Schritten in Apparate mit mechanischen Einbauten.

4. Verfahren nach Anspruch 3, **dadurch gekennzeichnet, dass** sich bei dem Verfahrensschritt um den Transport in Förderschnecken oder durch Zellradschleusen handelt.

5. Verfahren nach Anspruch 3, **dadurch gekennzeichnet, dass** der Verfahrensschritt in einem Pflugscharmischer, Schneckenmischer oder einem sonstigen Mischer mit mechanischen Misch- und/oder Zerhackwerkzeugen erfolgt.

6. Verfahren nach Anspruch 3, **dadurch gekennzeichnet, dass** der Verfahrensschritt in einer Nutsche, einem Pendelsieb, einem Rotationssieb oder einer anderen Filter- oder Siebeinrichtung mit mechanischen Werkzeugen erfolgt.

7. Verfahren nach Anspruch 3, **dadurch gekennzeichnet, dass** der Verfahrensschritt in einem Walzenbrecher, Stachel- oder Zahnwalzenbrecher Mühle, oder einem sonstigen Zerkleinerungsapparatur erfolgt.

8. Verfahren nach Anspruch 3, **dadurch gekennzeichnet, dass** der Verfahrensschritt in einem Schaufeltrockner, Tellertrockner oder Wirbelschichttrockner erfolgt.

9. Verfahren nach Anspruch 3, **dadurch gekennzeichnet, dass** eine Lagerung oder Zwischenpufferung Behälter ohne mechanische Werkzeuge erfolgt.

10. Verfahren nach Anspruch 3, **dadurch gekennzeichnet, dass** ein Transport in einem Transportbehälter erfolgt.
